# EUROPEAN PATENT APPLICATION

(11) **EP 1 094 080 A2**
(43) Date of publication of application: **25.04.2001**
(21) Application number: 00122904.6
(22) Date of filing: 20.10.2000
(51) Int. Cl.: C07K 16/40, C12N 5/20, C12P 21/08, G01N 33/573, G01N 33/577, C07K 1/22

(54) **Anti-dnase-gamma antibody, and production and use thereof**

(30) Priority: 21.10.1999 JP 29982499
(71) Applicant: Tanuma, Seiichi, Tokyo 192-03 (JP); Advanced Life Science Institute, Inc., Wako-shi, Saitama, 351-0112 (JP)
(72) Inventor: Tanuma, Seiichi, Hachioji-shi, Tokyo (JP); Shiokawa, Daisuke, Tokyo (JP); Kimura, Tatsuji, Advanced Life Science Inst., Inc., Wako-shi, Saitama 351-0112 (JP)
(74) Representative: Klunker . Schmitt-Nilson . Hirsch

(57) **Abstract**

Polyclonal antibodies and monoclonal antibodies specific for DNaseγ, hybridomas producing anti-DNaseγ monoclonal antibodies and an immunological detection method and purification method for DNaseγ using the antibodies are provided. By using a peptide containing the C-terminal portion sequence of the DNaseγ molecule as an immunogen it is possible to obtain anti-DNaseγ antibodies that specifically recognize DNaseγ. The DNaseγ monoclonal antibodies and polyclonal antibodies of the invention are characterized by specifically recognizing DNaseγ proteins.

## Description

### BACKGROUND OF INVENTION

### 1. Field of Invention

The present invention relates to specific polyclonal and monoclonal antibodies against the enzyme DNaseγ that is implicated in apoptosis, to hybridomas that produce the monoclonal antibodies and to an immunological detection method and purification method for DNaseγ using the antibodies.

### 2. Related Art

In recent years, apoptosis has been a major focus in the study of cell death. Unlike necrosis, which is passive cell death occurring suddenly due to burn or bruising, apoptosis is considered to be physiological and pathological cell death which is preprogrammed into the genes of the cell itself. That is, it is believed to be active cell death that occurs when triggering by some external or internal factor causes the gene programmed for apoptosis to be activated and the autodestruct program to be initiated.

Apoptosis is associated with a great many biological phenomena. Specifically, apoptosis has been implicated not only in morphogenesis during the development process but also in the case of mature individuals, in alternation of normal cells (removal of aged cells) such as epidermal cells of the skin and epithelial cells of the small intestine and stomach, atrophy due to glucocorticoids in the thymus which is a hormone-dependent tissue, atrophy of the prostate due to castration, as well as elimination of immunocompetent cells that react with self components or nerve cell death due to removal of neurotrophic factors.

Apoptosis is seen not only in such physiological cell death but also in pathological cell death of cells exposed to radiation or virus-infected cells. Cell death by apoptosis has also been reported as a cause of the reduction in T cells due to the AIDS virus, and is receiving attention for this reason. Apoptosis also occurs by chemical and physical stimuli such as administration of drugs or toxins, and heat. Apoptosis has also been clearly associated with nerve cell death in such neurodegenerative diseases as Alzheimer's, in natural elimination of tumor cells at cancer lesions and in cell death provoked by anticancer agents.

Consequently, elucidation of the molecular mechanism of apoptosis is important for gaining an understanding of the biological significance and physiological role played by cell death in the fundamental processes of life, including individual development, adult health maintenance and control (regulation) of oncogenesis.

The characteristic phenomena common to apoptosis are morphological changes such as cell contraction accompanying cell membrane alterations (loss of microvilli) and chromatin condensation, and fragmentation of chromatin DNA [Br. J. Cancer 26, 239-257(1972); Nature 284, 555-556(1980)]. Fragmentation of chromatin DNA into nucleosome units is the most notable phenomenon seen in all apoptotic cells regardless of the diversity of the factors responsible for its occurrence, and this suggests that the apoptosis cascade converges on the process of fragmentation of chromatin DNA.

The present inventors have discovered, isolated and purified a novel endonuclease DNaseγ that causes the DNA fragmentation characteristic of apoptosis. As a result of continuing research, it has been confirmed that DNaseγ is an endonuclease involved in the fragmentation of chromatin DNA during apoptosis, and the inventors have successfully determined and obtained the primary structure of the DNaseγ and the nucleotide sequence of its gene (Japanese Unexamined Patent Publication HEI No. 8-187079).

Since DNaseγ causes DNA fragmentation in apoptosis, elucidation of DNaseγ and its regulating mechanism is highly important in gaining an understanding of the overall molecular mechanism of apoptosis and the mechanism of cell survival and cell death. It will also be useful as a means of diagnosis, prevention and treatment development for diseases associated with apoptosis, such as cancer, autoimmune diseases, AIDS, neurodegenerative diseases such as Alzheimer's disease, and the like.

Upon further research, the present inventors have made additional discoveries. Specifically, it was found that DNaseγ expression is significantly lower in cancer lesion sites, and that almost no DNaseγ expression is seen in cancer cells such that apoptosis rarely occurs, but that introduction of the DNaseγ gene restores apoptosis ability. Furthermore, attention was focused on the fact that creation of antibodies for DNaseγ are useful since they can be utilized for diagnosis of the malignancy and prognosis for cancer. Attention was also directed to the fact that antibodies for DNaseγ are useful because they can also be utilized for analysis, diagnosis and treatment of apoptosis-related diseases including cancer, AIDS, autoimmune diseases and neurodegenerative diseases such as Alzheimer's disease.

### SUMMARY OF INVENTION

Highly specific antibodies provide a highly useful means for such analysis. By using highly specific antibodies it is possible to detect DNaseγ specifically and with high sensitivity, and measurement of DNaseγ can provide information useful for analysis and diagnosis of apoptosis-related conditions. It can also be used for purification of DNaseγ.

However, to date it has been difficult to obtain antibodies that are highly specific for DNaseγ. The present inventors have succeeded in fulfilling one of the conditions for obtaining such antibodies by isolating DNaseγ and elucidating the gene sequence and amino acid sequence thereof, thus making way for methods of producing antigen to be used for immunization. Generally speaking, if an antigen can be obtained with a certain degree of purity, it is possible to produce antibodies against it. However, production of antibodies is often difficult due to the properties of the antigen. With such antigens, it is necessary to study the conditions for obtaining such antibodies in light of general findings hitherto discovered, with additional research and trial-and-error on each of the antigens in order to obtain useful antibodies with high specificity and affinity.

It is therefore an object of the present invention to provide a method for production of antibodies that specifically recognize DNaseγ, as well as antibodies that specifically recognize DNaseγ and are useful for biochemical analysis and treatment and diagnosis of various diseases.

The present inventors have discovered a method for production of DNaseγ-specific antibodies by preparing different DNaseγ antigens and immunization and analysis. More precisely, it was found that it is difficult to produce specific antibodies by immunization with common recombinant proteins, and that antibodies for three different DNaseγ partial peptides do not recognize DNaseγ protein. However, it was found that it is possible to obtain DNaseγ protein-specific antibodies by using as the immunogen, a peptide corresponding to the C-terminal portion of DNaseγ.

In order to prepare specific antibodies for DNaseγ, the present inventors attempted to use various recombinant DNaseγ proteins and synthetic peptides as immunogens, based on the amino acid sequence for DNaseγ. It was found that when immunization is performed with DNaseγ protein, it is difficult to produce DNaseγ-specific antibodies since the antibody titer for DNaseγ does not increase sufficiently, and that using the three peptides other than the C-terminal portion as the immunogen yields antibodies that do not recognize the DNaseγ protein. As a result of diligent research it has been discovered that DNaseγ antibodies can be obtained by a useful method employing the C-terminal portion peptide of DNaseγ as the immunogen.

The present invention therefore provides antibodies that specifically recognizes DNaseγ protein. More particularly, it provides antibodies that specifically recognize peptides including the C-terminal amino acid sequence of DNaseγ protein. Even more particularly, it provides antibodies that specifically recognize peptides including the 12 amino acids: Val-Thr-Leu-Arg-Lys-Lys-Thr-Lys-Ser-Lys-Arg-Ser (SEQ ID No.1) of the C-terminus of human DNaseγ protein.

As concrete examples there may be mentioned monoclonal antibody hg302 produced by hybridoma hg302 (FERM P-17471) and monoclonal antibody hg303 produced by hybridoma hg303 (FERM P-17472).

The invention still further provides hybridomas that produce any of the antibodies described above. As concrete examples of such hybridomas there may be mentioned hybridoma hg302 (FERM P-17471) and hybridoma hg303 (FERM P-17472). The hybridoma hg302 was deposited at the National Institute of Bioscience and Human-Technology, 1-3, Higashi 1-chome Tsukuba-shi Ibaraki-ken 305-8566, Japan as FERM P-17471 on July 16, 1999 and transferred to an international deposition under the Budapest Treaty as FERM BP-7317 on October 12, 2000.

The hybridoma hg303 was deposited at the National Institute of Bioscience and Human-Technology, 1-3, Higashi 1-chome Tsukuba-shi Ibaraki-ken 305-8566, Japan as FERM P-17472 on July 16, 1999 and transferred to an international deposition under the Budapest Treaty as FERM BP-7318 on October 12, 2000.

The invention still further provides a method for production of any hybridoma, such as that described above, which is characterized by cloning cells obtained by fusion of spleen cells of a mammal immunized with a peptide containing a 5-20 amino acid sequence of the C-terminal portion of DNaseγ protein and myeloma cells of the same species. As a concrete example of such a sequence there may be mentioned the following amino acid sequence: Cys-Val-Thr-Leu-Arg-Lys-Lys-Thr-Lys-Ser-Lys-Arg-Ser (SEQ ID No.2).

The invention still further provides a method for immunological detection and measurement of DNaseγ, that comprises contacting an anti-DNaseγ antibody such as described above with a sample derived from a human or an animal believed to contain DNaseγ.

The invention still further provides a diagnostic reagent for prognosis of apoptosis-related conditions including cancer, AIDS, autoimmune diseases and neurodegenerative diseases such as Alzheimer's disease, which comprises an antibody such as described above.

The invention still further provides a method for purification of DNaseγ, which comprises contacting a solution containing DNaseγ with an affinity carrier prepared by binding an antibody such as described above to a high molecular carrier, for specific binding thereto, and then eluting the DNaseγ.

### BRIEF DESCRIPTION OF THE DRAWINGS

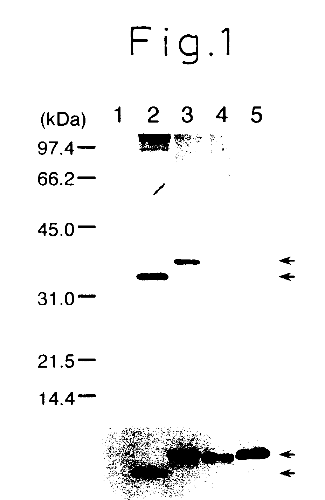

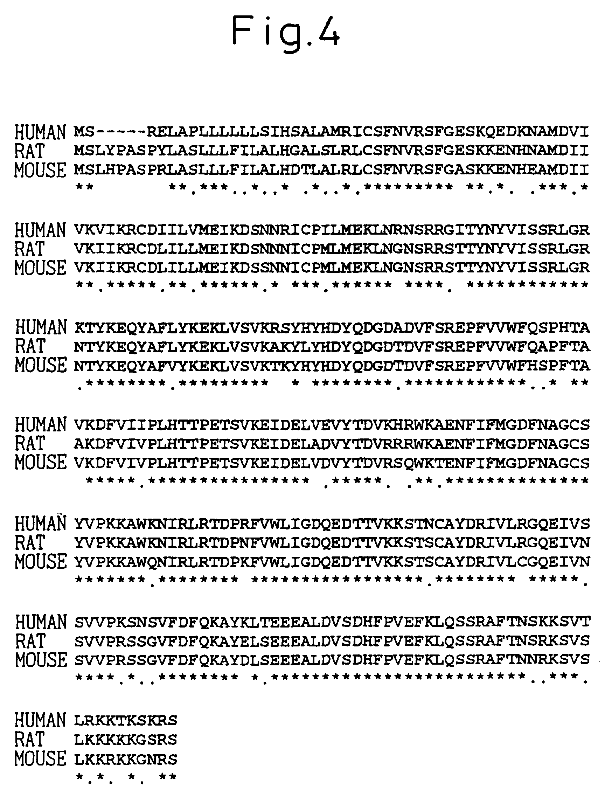

The upper panel in Fig. 1 is a Western blot image showing the reactivity between the monoclonal antibodies of the invention and DNaseγ of different species, and the lower panel is a photographic image showing the results of electrophoresis (zymography) for the enzyme activity of each DNaseγ (Example 2(2)).
   Lane 1 Negative control
   Lane 2 Recombinant human DNaseγ
   Lane 3 Recombinant human DNaseγ-Myc-His₆-tagged fusion
   Lane 4 Recombinant mouse DNaseγ-Myc-His₆-tagged fusion
   Lane 5 Recombinant rat DNaseγ-Myc-His₆-tagged fusion
Fig. 2 is a Western blot image from electrophoresis showing the reactivity between the monoclonal antibodies of the invention and different human-derived DNase proteins (Example 2(2)), with the results shown as a photographic image.
   Lane 1 Negative control
   Lane 2 Recombinant human DNaseγ-Myc-His₆-tagged fusion
   Lane 3 Recombinant human DNase I-Myc-His₆-tagged fusion
   Lane 4 Recombinant human DNase X-Myc-His₆-tagged fusion
   Lane 5 Recombinant human DNAS1L2-Myc-His₆-tagged fusion
Fig. 3 is an electrophoresis image (zymography) showing the results for immunoprecipitation of recombinant human DNaseγ using monoclonal antibodies hg303 of the invention or anti-Myc antibody, with the DNase activity shown as a photographic image.
   Lane 1: Precipitate of recombinant human DNaseγ-Myc-His₆ by immunoprecipitation using antibody hg303
   Lane 2: Supernatant of recombinant human DNaseγ-Myc-His₆ by immunoprecipitation using antibody hg303
   Lane 3: Precipitate of recombinant human DNaseγ-Myc-His₆ by immunoprecipitation using anti-Myc antibody
   Lane 4: Supernatant of recombinant human DNaseγ-Myc-His₆ by immunoprecipitation using anti-Myc antibody
   Lane 5: Precipitate of recombinant human DNaseγ by immunoprecipitation using antibody hg303
   Lane 6: Supernatant of recombinant human DNaseγ by immunoprecipitation using antibody hg303
   Lane 7: Precipitate of recombinant human DNaseγ by immunoprecipitation using antibody anti-Myc antibody
   Lane 8: Supernatant of recombinant human DNaseγ by immunoprecipitation using anti-Myc antibody
Fig. 4 shows a comparison between human, rat and mouse DNaseγ amino acid sequences (SEQ ID Nos. 3, 4, 5).

### EMBODIMENT OF CARRYING OUT THE INVENTION

An anti-DNaseγ antibody according to the invention is characterized by being an anti-DNaseγ antibody that specifically recognizes a peptide including the amino acid sequence of the C-terminal portion of the DNaseγ molecule. The anti-DNaseγ antibodies of the invention are characterized by being able to specifically recognize DNaseγ protein.

The amino acid sequence of the C-terminal portion of the DNaseγ molecule differs between species (Fig. 4: SEQ ID Nos. 3, 4 and 5), and therefore preparation of antibodies for this portion can yield antibodies with high species specificity. That is, by using the amino acid sequence of the C-terminal portion of human DNaseγ for immunization it is possible to obtain antibodies specific for human DNaseγ, and by using the amino acid sequence of the C-terminal portion of rat DNaseγ for immunization it is possible to obtain antibodies specific for rat DNaseγ. The method of the invention relates not only to anti-human DNaseγ antibodies but can also be applied to production of antibodies for DNaseγ of other animal species.

A peptide containing the amino acid sequence of the C-terminal portion of the DNaseγ molecule is defined as a peptide containing a sequence of at least 5 amino acids including the C-terminal of the DNaseγ molecule, and it also includes fused peptides with other peptides and proteins. It also includes these peptides bound to carrier proteins such as bovine serum albumin (BSA) and ovalbumin (OVA) for enhanced immunogenicity. The hybridomas of the invention are hybridomas having the ability to produce monoclonal antibodies that can specifically recognize the C-terminal sequence of DNaseγ and can specifically recognize DNaseγ protein.

The aforementioned peptide comprising at least 5 amino acid residues and preferably 5-20 amino acid residues may be used as an immunogen to obtain antibodies. It is common to employ a method in which the peptide is chemically bound to a carrier protein such as KLH, BSA or OVA for increased immunogenicity. For the chemical bonding, peptides including cysteine at the binding sites may be constructed and the -SH group of cysteine used for binding with the carrier protein. When a peptide of the C-terminal portion is used, it is common to bind the carrier protein at a cysteine at the N-terminal of the peptide.

Any of various chemical crosslinking agents may be used for the chemical binding of the peptide and carrier protein. A crosslinking agent with appropriate reactivity with the target functional group is selected. For example, for crosslinking between an amino group and -SH group, succinimidyl-4-[N-maleimidomethyl]cyclohexane-1-carboxylate (SMCC) or N-succinimidyl-3-[2-pyridyldithio]propionate (SPDP) or the like is used.

Guinea pigs, rats, mice or the like may be used to obtain the monoclonal antibodies, but mice are preferred for simplicity of production and procedure. The immunization method may be by any route such as intraabdominally, subcutaneously, intravenously, intramuscularly or intradermally for immunization of a mouse, for example, but intraabdominal, subcutaneous or intravenous injection are primarily preferred. Numerous methods with high variability in terms of immunization interval and immunization dose are possible, but common methods use spleen cells extracted 1-5 days and preferably about 2-4 days after final immunization of about 2-6 immunizations at 2-week intervals, for example.

The immunization dose is about 0.1 µg or greater and preferably about 1-30 µg per mouse, as the single peptide amount. Before extraction of the spleens, it is preferred to conduct a fusion experiment using the spleen cells after partial blood collection to confirm blood antibody titer increase. Cell fusion between the spleen cells and lymphoid cells is accomplished by fusing the extracted mouse spleen cells with an appropriate lymphoid cell line such as myeloma (e.g., SP2, P3-X63-Ag·8UI) from the same species or a different species (preferably the same species) using a hypoxanthine-guanine-phosphorybosyl transferase negative (HGPRT⁻) or thymidine kinase negative (TK⁻) marker.

These may be produced by fusion according to the lane method, for example (Methods in Enzymology, 121, 1980; 183-192). That is, myeloma cells and spleen cells, for example, are suspended at a proportion of about 3:5 in RPMI 1640 medium, for example, and a fusing agent such as polyethylene glycol (PEG) or Sendai virus is used. The polymerization degree of the PEG is normally about 1000-6000, the time is about 0.5-30 minutes and the concentration is 10-80%; however, efficient fusion can be achieved by treatment using PEG 1500 at about 35% for about 4-10 minutes, as a preferred example of conditions.

The cell fusion may be carried out by selective and efficient growth using hypoxanthine-azaserine medium (HA medium). The cell culture supernatant resulting from the growth may be screened to determine whether the target antibodies have been produced, and the antibody titer screening may be carried out in the following manner. That is, the presence of production of antibodies against the immunized peptide may be examined by a method such as Enzyme ImmunoAssay (EIA) or Radio ImmunoAssay (RIA), and various modifications are possible within these methods.

One method based on EIA will be examined as a preferred assay method. An immunized peptide is coated onto a 96-well microtest plate by a common method, and the culture supernatant or mouse serum to be measured is added and reacted therewith for a fixed time at a constant temperature (approximately 4-40°C, same hereunder). After then thoroughly washing the reaction product, enzyme-labeled anti-mouse immunoglobulin antibody, for example, (obtained by coupling of the enzyme and antibody by a common method followed by purification) is added for reaction for a fixed time at a constant temperature. After thoroughly washing the reaction product, the enzyme substrate is added for reaction for a fixed time at a constant temperature, and then the color produced is measured by absorbance or fluorescence.

The cells in the wells in which growth in the selective medium was observed and antibody activity was detected for the peptide used for immunization, are preferably cloned by the limiting dilution method or a similar method. The supernatant of the cloned cells is screened in the same manner and the cells in the wells with high antibody activity are increased to obtain monoclonal antibody-producing hybridoma clones exhibiting reactivity with the immunized peptide. The hybridomas cloned in this manner may be grown in liquid medium to obtain the monoclonal antibodies from the culture solution.

The antibodies can also be obtained by intraabdominally inoculating a mammalian animal, allowing the cells to proliferate and collecting the ascitic fluid. For this purpose, in the case of mice for example, BALB/c mice previously inoculated with pristane (tetramethylpentadecane), mineral oil or the like are then intraabdominally inoculated with approximately 1 x 10⁴ - 1 x 10⁷, and preferably approximately 5 x 10⁵ - 2 x 10⁶ hybridoma cells, and after about 7-20 days and preferably about 10-14 days, the ascitic fluid is collected. The antibodies accumulated in the ascitic fluid can be obtained by easily isolating the monoclonal antibodies as pure immunoglobulins by, for example, ammonium sulfate precipitation, protein A affinity chromatography, or the like.

The method for immunological detection of DNaseγ according to the invention is characterized by contacting the monoclonal antibodies with a sample containing tissue, body fluid or a cell line from a human or animal believed to contain DNaseγ, to detect the DNaseγ.

Immunochemical detection methods that may be applied for the invention include, for example, immunohistostaining, Western blotting, agglutination reaction, agglutination inhibition, immunodiffusion, immunonephelometry, latex nephelometry, competitive assay (solid phase method, second antibody method), sandwich assay, immunometric assay, liposome immunolysis assay (LILA) or the like, and detection methods using labeling substances include radioimmunoassay (RIA), enzyme immunoassay (EIA), fluorescent immunoassay (FIA), chemiluminescent immunoassay, metal immunoassay, spin immunoassay and the like.

The present invention provides a method for production of specific antibodies for DNaseγ. According to the invention, immunization is carried out with an amino acid sequence specific for DNaseγ protein, and therefore DNaseγ-specific antibodies are obtained that do not cross-react with other DNase proteins such as DNaseI and DNaseX. Also, because the amino acid sequence of the C-terminal portion of DNaseγ is less conserved among animal species (Fig. 4), antibodies for this portion have high species specificity. In other words, anti-human DNaseγ antibodies of the invention do not cross-react with DNaseγ of other species. The anti-DNaseγ antibody production method of the invention is not limited to animal species. It may be applied to production of antibodies for human DNaseγ as well as for DNaseγ of other species.

The antibodies of the invention allow specific and convenient detection of DNaseγ protein, and therefore provide a means of analyzing the function and pathological significance of DNaseγ protein in the field of medical biology. In addition, since DNaseγ protein is a DNase associated with apoptosis and DNaseγ expression decreases with canceration or malignant degeneration, the antibodies of the invention can be used in the medical and pharmaceutical industry as agents for research, diagnosis and treatment of apoptosis-related conditions including cancer, AIDS, autoimmune diseases and neurodegenerative diseases such as Alzheimer's disease. The antibodies of the invention are also useful for analysis of conditions using animal models for apoptosis-related diseases. This is expected to be of use in predicting and detecting apoptosis sensitivity and particularly to be of use in the analysis, treatment and diagnosis of conditions related to apoptosis.

### EXAMPLES

The present invention will now be explained in more detail by way of examples and reference examples which, however, are in no way intended to restrict the invention.

### Example 1. Production of monoclonal antibodies

### Preparation of human DNaseγ peptide antigen

Peptide hg3 (Cys-Val-Thr-Leu-Arg-Lys-Lys-Thr-Lys-Ser-Lys-Arg-Ser) (SEQ ID No.2) comprising 13 amino acids including a 12 amino acid sequence corresponding to the C-terminal portion of human DNaseγ, was chemically synthesized by the Fmoc method and was subjected to salting out and purification.

Bovine serum albumin (BSA) and ovalbumin (OVA) were dissolved in a 0.1 M phosphate buffer solution (pH 7.0) as carrier proteins, 50 molar equivalents of SMCC (Pierce Co.) were added and reacted with the amino groups of the carrier proteins for introduction of NHS groups, and the unreacted components were removed by gel filtration. The peptide hg3 was dissolved in 0.5 mM EDTA and 0.1 M phosphate buffer solution (pH 6.0) and 50 molar equivalents of peptide was mixed with the carrier protein into which NHS groups had been introduced, for reaction with the SH groups of the peptide. The unreacted components were removed by dialysis to yield hg3-BSA, hg3-OVA conjugates.

### Preparation of hybridomas

### (1) Immunization

The hg3-BSA was administered through the abdomen of BALB/c mice with Freund's complete adjuvant (FCA), at 20 µg per mouse. After 2, 4, 7 and 9 weeks, a booster immunization of 5-10 µg of hg3-BSA was administered through the abdomen of the mice with Freund's incomplete adjuvant (FIA).

### (2) Cell fusion

A 5 µg portion of hg3-BSA was intravenously administered to the immunized mice, and after 3 days from administration the spleens were extracted and the spleen cells obtained. A common method was applied for cell fusion of the spleen cells with the mouse myeloma cell line SP2/0 Ag14 using polyethylene glycol, and then HAT medium was used for selective growth of the hybrid cells.

### (3) Screening and cloning of antibody-producing cells

hg3-OVA was first immobilized on a 96-well polystyrene microtest plate, the culture supernatant of the hybrid cells was reacted therewith, and primary screening was performed for anti-human DNaseγ antibody-producing cells by solid phase enzyme-linked immunoassay (ELISA) using HRP-labeled goat anti-mouse IgG antibody (Jackson Immune Research). A secondary screening was then performed by ELISA in the same manner using a plate with recombinant human DNaseγ in solid phase.

Next, the human DNaseγ protein-specific antibody-producing wells were selected and the cells were cloned by the limiting dilution method. As a result, two different hybridomas were obtained and were designated as hybridoma hg302 and hg303, respectively.

The hybridomas hg302 and hg303 have been deposited at the National Institute of Bioscience and Human Technology don July 16, 1999 as FERM P-17471 and FERM P-17472, respectively.

### Purification and immunoglobulin classification of monoclonal antibodies

The mouse hybridoma cells producing monoclonal antibodies specific for human DNaseγ protein were intraabdominally inoculated into mature BALB/c mice that had already been intraabdominally administered pristane (tetramethylpentadecane), and after 10-14 days the abdomens of the mice that had enlarged due to proliferation of the hybridomas were dissected and the ascites fluid was collected. The antibodies were purified from the collected ascitic fluid using a HiTrap Protein A Column (Pharmacia).

The immunoglobulin class of the purified antibodies was determined using a mouse monoclonal antibody subclass ID kit (Zymed). As a result, the anti-human DNaseγ protein monoclonal antibodies hg302 and hg303 were both found to be IgG1, κ.

### Example 2. Property analysis of monoclonal antibodies

The specificity and reactivity of the antibodies of the invention are extremely high, and they can therefore be used for Western blotting, immunoprecipitation, immunostaining and the like, as well as for analysis of DNA cleaving and apoptosis, which are the functions of human DNaseγ protein. Examples are given below. Figs. 1-3 show the results of using hg303 monoclonal antibody for illustration, but similar results are obtained with hg302 monoclonal antibody as well.

### (1) Preparation of antigen protein-expressing cells to be used for analysis.

The following primer pair was used for amplification of an open reading frame of DNaseγ containing the termination codon by PCR from cDNA constructed using spleen-derived polyA(+)RNA as the template, and this was followed by subcloning in pBluescript II KS+.
DNaseγ sense primer: GCTAGCCACCATGTCACGGGAGCTGGCC (SEQ ID No:6)
DNaseγ antisense primer 1: GCTAGCCTAGGAGCGTTTGCTCTTTG (SEQ ID No:7) (with linker sequence containing NheI recognition site (GCTAGC) added to 5' end)

After confirming the sequences by cycle sequencing, the DNaseγ in-site DNA cut out by NheI digestion was subcloned at the XBaI site of pcDNA3.1-Myc-His B (Invitrogen Co.), to obtain the wild type DNaseγ-coding expression vector phDNaseγ.

The following primer pair was used for amplification of the open reading frames of human DNaseγ, DNase I, DNase X and DNASIL2 minus the termination codon by PCR from cDNA constructed using spleen-derived, placenta-derived, GOTO cell-derived and HeLa cell-derived polyA(+)RNA as the template, respectively, and this was followed by subcloning in pBluescript II KS+.
DNaseγ sense primer: GCTAGCCACCATGTCACGGGAGCTGGCC (SEQ ID No:6)
DNaseγ antisense primer 2: GCTAGCGAGCGTTTGCTCTTTG (SEQ ID No:8) (with linker sequence containing NheI recognition site (GCTAGC) added to 5' end)
DNase I sense primer: CTCGAGCCACCATGAGGGGCATGAAGCTGCTG (SEQ ID No:9)
DNase I antisense primer: CTCGAGACTTCAGCATCACCTCCACTG (SEQ ID No:10)
DNase X sense primer: CTCGAGCCACCATGCACTACCCAACTGCAC (SEQ ID No:11)
DNase X antisense primer: CTCGAGAGGCAGCAGGGCACAGCTGA (SEQ ID No:12)
DNAS1L2 sense primer: CTCGAGCCACCATGGGCGGGCCCCGGGCT (SEQ ID No:13)
DNAS1L2 antisense primer: CTCGAGATCGGTGGAACTTGAGGGTCAC (SEQ ID No:14) (with linker sequence containing XhoI recognition site (CTCGAG) added to each at 5' end)

After confirming the sequences by cycle sequencing, the DNaseγ in-site DNA cut out by NheI digestion was subcloned at the XBaI site of pcDNA3.1-Myc-His B (Invitrogen Co.), to obtain the DNaseγ-coding expression vector phDNaseγ-Myc-His, containing Myc and histidine tags at the C-terminus. The in-site DNA cut out by XhoI digestion was recloned at the XhoI site of pcDNA3.1-Myc-His C (Invitrogen Co.), to obtain the DNase I-, DNase X-and DNASIL2-coding expression vectors phDNase I-Myc-His, phDNase X-Myc-His, phDNAS1L2-Myc-His, containing Myc and histidine tags at the C-terminals.

These expression vectors were each used for transfection of 1 x 10⁶ HeLa S3 cells, and after 39 hours of culturing, cells were collected which expressed human DNaseγ and human, mouse and rat DNaseγ-Myc-His₆-tagged fusion proteins, and the protein analogues human DNase I-Myc-His₆, DNase X-Myc-His₆ and DNAS1L2-Myc-His₆.

### (2) Reactivity of DNaseγ of each animal species by Western blotting

The cells expressing the human DNaseγ- and human, mouse and rat DNaseγ-Myc-His₆-tagged fusion proteins were subjected to SDS-polyacrylamide gel electrophoresis (SDS-PAGE) at 2 x 10⁵ cells/lane. The protein in the gel was transferred to a nitrocellulose membrane, and the membrane was subjected to blocking with 2.5% dry milk and then reacted for one hour at room temperature with the anti-human DNaseγ monoclonal antibodies hg302 and hg303. After rinsing, it was further reacted for one hour at room temperature with alkaline phosphatase-labeled anti-mouse IgG (NEW ENGLAND BioLabs) and rinsed, and then the coloration was detected using BCIP/NBT as the substrate.

The upper panel in Fig. 1 shows the reaction of anti-DNaseγ monoclonal antibody hg303.
Lane 1 Negative control
Lane 2 Recombinant human DNaseγ
Lane 3 Recombinant human DNaseγ-MycHis₆-tagged fusion
Lane 4 Recombinant mouse DNaseγ-MycHis₆-tagged fusion
Lane 5 Recombinant rat DNaseγ-MycHis₆-tagged fusion

This demonstrates that the antibodies of the invention react specifically with human DNaseγ protein of molecular weight 33 kDa and with the human DNaseγ-Myc-His₆ fusion protein of 36 kDa, but do not cross-react with mouse and rat DNaseγ. That is, high species specificity was exhibited by the antibodies of the invention.

1 x 10⁵ cells/lane of each of the protein-expressing cells were subjected to SDS-PAGE with dsDNA containing gel. The gel was rinsed for one hour at 50°C in a solution containing 5 mM 2-mercaptoethanol (2-ME) and 10 mM Tris-HCl (pH 7.8), and then the protein in the gel was subjected to refolding at 4°C for 3 hours in a solution containing 10 mM Tris-HCl (pH 7.8). The gel was transferred into a solution containing 1 mM 2-ME, 3 mM CaCl₂, 3 mM MgCl₂ and 10 mM Tris-HCl (pH 7.8), for degradation reaction of the dsDNA with DNase at 37°C for 10 hours. Fluorescent staining was performed with ethidium bromide, and the remaining dsDNA was detected. The portions of dsDNA degradation by the DNase appear black.

The results are shown in the lower panel of Fig. 1. Roughly the same enzyme activity is seen in lanes 2-5, indicating approximately the same amounts of DNase protein.

### (3) Reactivity with DNaseγ analog proteins by Western blotting

The cells expressing the human DNaseγ-Myc-His₆-, DNase I-Myc-His₆-, DNase X-Myc-His₆- and DNAS1L2-Myc-His₆-tagged fusion proteins were subjected to SDS-PAGE at 2 x 10⁵ cells/lane. The protein in the gel was transferred to a nitrocellulose membrane, and the membrane was subjected to blocking with 2.5% dry milk and then reacted for one hour at room temperature with the anti-human DNaseγ monoclonal antibodies hg302 and hg303. After rinsing, it was further reacted for one hour at room temperature with alkaline phosphatase-labeled anti-mouse IgG (NEW ENGLAND BioLabs) and rinsed, and then the coloration was detected using BCIP/NBT as the substrate.

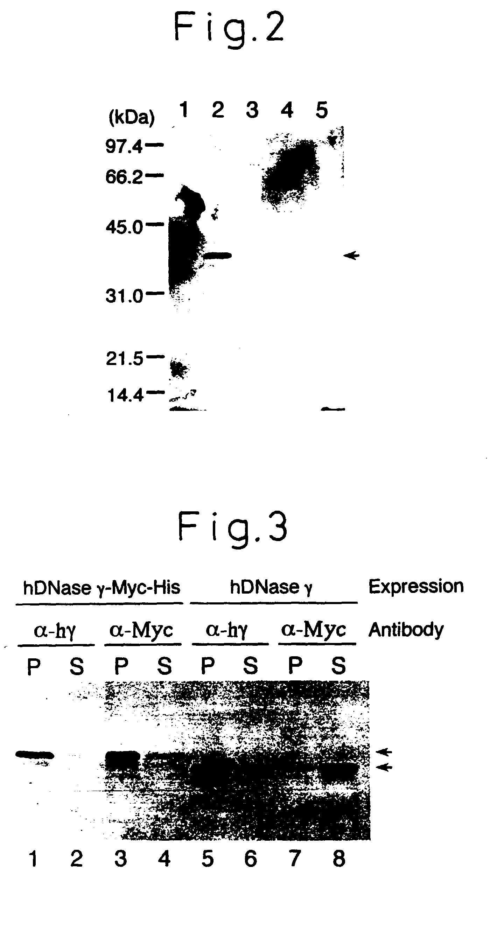

The results with hg303 monoclonal antibody are shown in Fig. 2.
Lane 1 Negative control
Lane 2 Recombinant human DNaseγ-Myc-His₆-tagged fusion
Lane 3 Recombinant human DNase I-Myc-His₆-tagged fusion
Lane 4 Recombinant human DNase X-Myc-His₆-tagged fusion
Lane 5 Recombinant human DNAS1L2-Myc-His₆-tagged fusion

Fig. 2 shows that the antibodies of the invention react specifically with the human DNaseγ-Myc-His₆ fusion protein of molecular weight 36 kDa, but do not cross-react with the analog proteins DNase I, DNase X and DNASIL2 that are homologous with DNaseγ.

### (4) Reactivity by immunoprecipitation

Cells expressing human DNaseγ and human DNaseγ-Myc-His₆-tagged fusion proteins were dissolved in 0.3% NP-40 solution at 1 x 10⁵ cells, and then the anti-human DNaseγ monoclonal antibodies hg302, hg303 or anti-Myc antibody (Invitrogen) were added to the supernatant after centrifugal separation, for reaction at 4°C for 90 minutes. Protein G Sepharose FF was added at 1/10 the volume of the cell extract and then further reaction for 60 minutes was followed by centrifugation to obtain a supernatant (S). The precipitate was rinsed 3 times with NP-40 solution and dissolved in an SDS-PAGE sample buffer, to obtain a precipitate (P). Both S and P were applied to a gel containing dsDNA at approximately 1 x 10⁵ cells/lane, and after SDS-PAGE the DNA degradating activity was analyzed in the same manner as above.

Fig. 3 shows the results of immunoprecipitation with hg303 monoclonal antibody.
Lane 1: Precipitate of recombinant human DNaseγ-Myc-His₆ by immunoprecipitation using antibody hg303
Lane 2: Supernatant of recombinant human DNaseγ-Myc-His₆ by immunoprecipitation using antibody hg303
Lane 3: Precipitate of recombinant human DNaseγ-Myc-His₆ by immunoprecipitation using anti-Myc antibody
Lane 4: Supernatant of recombinant human DNaseγ-Myc-His₆ by immunoprecipitation using anti-Myc antibody
Lane 5: Precipitate of recombinant human DNaseγ by immunoprecipitation using antibody hg303
Lane 6: Supernatant of recombinant human DNaseγ by immunoprecipitation using antibody hg303
Lane 7: Precipitate of recombinant human DNaseγ by immunoprecipitation using antibody anti-Myc antibody
Lane 8: Supernatant of recombinant human DNaseγ by immunoprecipitation using anti-Myc antibody

Fig. 3 shows that human DNaseγ precipitates with anti-human DNaseγ hg303 antibody but not with anti-Myc antibody, while human the DNaseγ-Myc-His₆-tagged fusion protein precipitates with anti-human DNaseγ hg303 antibody and anti-Myc antibody.

The results indicated that the anti-human DNaseγ hg303 antibody also reacts with human DNaseγ under solution conditions.

### Reference Example 1. Preparation of recombinant human DNaseγ

Human DNaseγ cDNA was incorporated into a thioredoxin (Trx)-histidine-tagged fusion protein expression vector (pET32a, Novagene) and a glutathione S transferase (GST) fusion protein expression vector (pGEX3X, Pharmacia), and then Trx-DNaseγ was expressed in *E*. *coli* strain AD494(DE3) and GST-DNaseγ was expressed in strain DH5α. The *E*. *coli* were cultured and the cells were collected by centrifugation. The collected cells were lysed and then the respective recombinant proteins were purified by affinity chromatography using a cobalt-binding column and a glutathione-binding column.

### Reference Example 2. Immunization with recombinant human DNaseγ

A 20 µg portion of the aforementioned Trx-DNaseγ and GST-DNaseγ was intraabdominally administered to BALB/c mice with FCA. At 2 and 4 weeks after administration, 5-10 µg was intraabdominally administered as a booster with FIA to the mice. One week after the final immunization, when blood was sampled from the tail and the antibody titer thereof measured by antigen solid phase ELISA, the antibody titer for Trx and GST were both higher but no increase was found in the antibody titer for DNaseγ. It was thus demonstrated that production of anti-DNaseγ antibodies is difficult by immunization of common recombinant proteins.

### Reference Example 3. Synthesis of hg1, hg2 #1740 and immunization

The Fmoc method was used for chemical synthesis followed by salting out and purification of peptide hg1 (SEQ ID No. 15) comprising 12 amino acids including an 11 amino acid sequence corresponding to human DNaseγ (76-86), peptide hg2 (SEQ ID No. 16) comprising 11 amino acids including a 10 amino acid sequence corresponding to human DNaseγ (139-148) and peptide #1740 (SEQ ID No. 17) comprising 10 amino acids including a 9 amino acid sequence corresponding to human DNaseγ (79-87).

Bovine serum albumin (BSA) and ovalbumin (OVA) were reacted with SMCC (Pierce) to introduce NHS groups. After removing the unreacted components, the SH groups of the aforementioned peptides hg1, hg2 and #1740 were reacted therewith to construct the conjugates hg1-BSA, hg1-OVA, hg2-BSA, hg2-OVA, #1740-BSA and #1740-OVA. A 20 µg portion each of hg1-BSA, hg2-BSA and #1740-BSA was intraabdominally administered with FCA to BALB/c mice.

After 2 and 4 weeks, 10 µg each of hg1-BSA, hg2-BSA and #1740-BSA was intraabdominally given to the mice with Freund's incomplete adjuvant (FIA) as a booster immunization. One week after the final immunization, blood was sampled from the tail and the antibody titer thereof measured by antigen solid phase ELISA. The antibody titers for hg1-OVA, hg2-OVA and #1740-OVA, i.e. the antibody titers for the peptides, were higher but no increase was found in the antibody titer for DNaseγ protein. It was thus demonstrated that antibodies for peptides hg1, hg2 and #1740 cannot recognize DNaseγ protein.

## Claims

1. An antibody that specifically recognizes DNaseγ protein.

2. An antibody according to claim 1 that specifically recognizes a peptide including the C-terminal amino acid sequence of DNaseγ protein.

3. An antibody according to claim 1 or 2 that specifically recognizes a peptide including the 12 amino acids: Val-Thr-Leu-Arg-Lys-Lys-Thr-Lys-Ser-Lys-Arg-Ser (SEQ ID No.1) of the C-terminus of human DNaseγ protein.

4. An antibody according to any one of claims 1 to 3, which is monoclonal antibody hg302 produced by hybridoma hg302 (FERM P-17471) or monoclonal antibody hg303 produced by hybridoma hg303 (FERM P-17472).

5. A hybridoma that produces an antibody according to any one of claims 1 to 4.

6. A hybridoma according to claim 5, which is hybridoma hg302 (FERM P-17471) or hybridoma hg303 (FERM P-17472).

7. A method for production of a hybridoma according to claim 5, characterized by cloning cells obtained by fusion of spleen cells of a mammal immunized with a peptide containing a 5-20 amino acid sequence of the C-terminal portion of DNaseγ protein and myeloma cells of the same species.

8. The method of claim 7, wherein said amino acid sequence of the C-terminal portion is the following sequence: Cys-Val-Thr-Leu-Arg-Lys-Lys-Thr-Lys-Ser-Lys-Arg-Ser (SEQ ID No.2).

9. A method for immunological detection and measurement of DNaseγ, that comprises contacting anti-DNaseγ antibody according to any one of claims 1 to 4 with a sample derived from a human or animal believed to contain DNaseγ.

10. A diagnostic reagent for prognosis of apoptosis-related conditions including cancer, AIDS, autoimmune diseases and neurodegenerative diseases such as Alzheimer's disease, which comprises an antibody according to any one of claims 1 to 4.

11. A method for purification of DNaseγ, which comprises contacting a solution containing DNaseγ with an affinity carrier prepared by binding an antibody according to any one of claims 1 to 4 to a high molecular carrier, for specific binding thereto, and then eluting the DNaseγ.


SEQUENCE LISTING
<110> Advanced Life Science Institute, Inc.
<120> Anti-DNase gamma antibody, as well as production and
      use thereof
<130> 993721
<160>
<210> 1
<211> 12
<212> PRT
<213> Artificial Sequence
<220>
<221>
<223> A part of human DNase gamma
<400> 1
Val Thr Leu Arg Lys Lys Thr Lys Ser Lys Arg Ser
 1               5                   10

<210> 2
<211> 13
<212> PRT
<213> Artificial Sequence
<220>
<221>
<223> A part of human DNase gamma
<400> 2
Cys Val Thr Leu Arg Lys Lys Thr Lys Ser Lys Arg Ser
 1               5                   10

<210> 3
<211> 305
<212> PRT
<213> Homo sapiens
<223> Amino acid sequence of human DNase gamma
<400> 3

<210> 4
<211> 310
<212> PRT
<213> Rat
<223> Amino acid sequence of rat DNase gamma
<400> 4
Met Ser Leu Tyr Pro Ala Ser Pro Tyr Leu Ala Ser Leu Leu Leu Phe
1               5                   10                  15
Ile Leu Ala Leu His Gly Ala Leu Ser Leu Arg Leu Cys Ser Phe Asn
            20                  25                  30
Val Arg Ser Phe Gly Glu Ser Lys Lys Glu Asn His Asn Ala Met Asp
        35                  40                  45
Ile Ile Val Lys Ile Ile Lys Arg Cys Asp Leu Ile Leu Leu Met Glu
    50                  55                  60
Ile Lys Asp Ser Asn Asn Asn Ile Cys Pro Met Leu Met Glu Lys Leu
65                  70                  75                  80
Asn Gly Asn Ser Arg Arg Ser Thr Thr Tyr Asn Tyr Val Ile Ser Ser
                85                  90                  95
Arg Leu Gly Arg Asn Thr Tyr Lys Glu Gln Tyr Ala Phe Leu Tyr Lys
            100                 105                 110
Glu Lys Leu Val Ser Val Lys Ala Lys Tyr Leu Tyr His Asp Tyr Gln
        115                 120                 125
Asp Gly Asp Thr Asp Val Phe Ser Arg Glu Pro Phe Val Val Trp Phe
    130                 135                 140
Gln Ala Pro Phe Thr Ala Ala Lys Asp Phe Val Ile Val Pro Leu His
145                 150                 155                 160
Thr Thr Pro Glu Thr Ser Val Lys Glu Ile Asp Glu Leu Ala Asp Val
                165                 170                 175
Tyr Thr Asp Val Arg Arg Arg Trp Lys Ala Glu Asn Phe Ile Phe Met
            180                 185                 190
Gly Asp Phe Asn Ala Gly Cys Ser Tyr Val Pro Lys Lys Ala Trp Lys
        195                 200                 205
Asn Ile Arg Leu Arg Thr Asp Pro Asn Phe Val Trp Leu Ile Gly Asp
    210                 215                 220
Gln Glu Asp Thr Thr Val Lys Lys Ser Thr Ser Cys Ala Tyr Asp Arg
225                 230                 235                 240
Ile Val Leu Arg Gly Gln Glu Ile Val Asn Ser Val Val Pro Arg Ser
                245                 250                 255

Ser Gly Val Phe Asp Phe Gln Lys Ala Tyr Glu Leu Ser Glu Glu Glu
            260                 265                 270
Ala Leu Asp Val Ser Asp His Phe Pro Val Glu Phe Lys Leu Gln Ser
        275                 280                 285
Ser Arg Ala Phe Thr Asn Ser Arg Lys Ser Val Ser Leu Lys Lys Lys
    290                 295                 300
Lys Lys Gly Ser Arg Ser
305                 310

<210> 5
<211> 310
<212> PRT
<213> Mouse
<223> Amino acid sequence of mouse DNase gamma
<400> 5
Met Ser Leu His Pro Ala Ser Pro Arg Leu Ala Ser Leu Leu Leu Phe
1               5                   10                  15
Ile Leu Ala Leu His Asp Thr Leu Ala Leu Arg Leu Cys Ser Phe Asn
            20                  25                  30
Val Arg Ser Phe Gly Ala Ser Lys Lys Glu Asn His Glu Ala Met Asp
        35                  40                  45
Ile Ile Val Lys Ile Ile Lys Arg Cys Asp Leu Ile Leu Leu Met Glu
    50                  55                  60
Ile Lys Asp Ser Ser Asn Asn Ile Cys Pro Met Leu Met Glu Lys Leu
65                  70                  75                  80
Asn Gly Asn Ser Arg Arg Ser Thr Thr Tyr Asn Tyr Val Ile Ser Ser
                85                  90                  95
Arg Leu Gly Arg Asn Thr Tyr Lys Glu Gln Tyr Ala Phe Leu Tyr Lys
            100                 105                 110
Glu Lys Leu Val Ser Val Lys Thr Lys Tyr His Tyr His Asp Tyr Gln
        115                 120                 125
Asp Gly Asp Thr Asp Val Phe Ser Arg Glu Pro Phe Val Val Trp Phe
    130                 135                 140
His Ser Pro Phe Thr Ala Val Lys Asp Phe Val Ile Val Pro Leu His
145                 150                 155                 160
Thr Thr Pro Glu Thr Ser Val Lys Glu Ile Asp Glu Leu Val Asp Val
                165                 170                 175
Tyr Thr Asp Val Arg Ser Gln Trp Lys Thr Glu Asn Phe Ile Phe Met
            180                 185                 190

Gly Asp Phe Asn Ala Gly Cys Ser Tyr Val Pro Lys Lys Ala Trp Gln
        195                 200                 205
Asn Ile Arg Leu Arg Thr Asp Pro Lys Phe Val Trp Leu Ile Gly Asp
    210                 215                 220
Gln Glu Asp Thr Thr Val Lys Lys Ser Thr Ser Cys Ala Tyr Asp Arg
225                 230                 235                 240
Ile Val Leu Cys Gly Gln Glu Ile Val Asn Ser Val Val Pro Arg Ser
                245                 250                 255
Ser Gly Val Phe Asp Phe Gln Lys Ala Tyr Asp Leu Ser Glu Glu Glu
            260                 265                 270
Ala Leu Asp Val Ser Asp His Phe Pro Val Glu Phe Lys Leu Gln Ser
        275                 280                 285
Ser Arg Ala Phe Thr Asn Asn Arg Lys Ser Val Ser Leu Lys Lys Arg
    290                 295                 300
Lys Lys Gly Asn Arg Ser
305                 310

<210> 6
<211> 28
<212> DNA
<213> Artificial Sequence
<220>
<223> DNase gamma sense primer
<400> 6
gctagccacc atgtcacggg agctggcc 28

<210> 7
<211> 26
<212> DNA
<213> Artificial Sequence
<220>
<223> DNase gamma antisense primer 1
<400> 7
gctagcctag gagcgtttgc tctttg 26

<210> 8
<211> 22
<212> DNA

<213> Artificial Sequence
<220>
<223> DNase gamma antisence primer 2
<400> 8
gctagcgagc gtttgctctt tg                                                22

<210> 9
<211> 32
<212> DNA
<213> Artificial Sequence
<220>
<223> DNase 1 sense primer
<400> 9
ctcgagccac catgaggggc atgaagctgc tg                                     32

<210> 10
<211> 27
<212> DNA
<213> Artificial Sequence
<220>
<223> DNase 1 antisense primer
<400> 10
ctcgagactt cagcatcacc tccactg                                           27

<210> 11
<211> 30
<212> DNA
<213> Artificial Sequence
<220>
<223> DNase X sense primer
<400> 11
ctcgagccac catgcactac ccaactgcac                                        30

<210> 12
<211> 26
<212> DNA
<213> Artificial Sequence

<220>
<223> DNase X antisense primer
<400> 12
ctcgagaggc agcagggcac agctga 26

<210> 13
<211> 29
<212> DNA
<213> Artificial Sequence
<220>
<223> DNAS1L2 sense primer
<400> 13
ctcgagccac catgggcggg ccccgggct 29

<210> 14
<211> 28
<212> DNA
<213> Artificial Sequence
<220>
<223> DNAS1L2 antisense primer
<400> 14
ctcgagatcg gtggaacttg agggtcac 28

<210> 15
<211> 12
<212> PRT
<213> Artificial Sequence
<220>
<221>
<222>
<223> A part of human DNase gamma
<400> 15
Asn Arg Asn Ser Arg Arg Gly Ile The Tyr Asn Cys
1 5 10

<210> 16
<211> 11

<212> PRT
<213> Artificial Sequence
<220>
<221>
<222>
<223> A part of human DNase gamma
<400> 16
Phe Gln Ser Pro His Thr Ala Val Lys Asp Cys
1               5                   10

<210> 17
<211> 10
<212> PRT
<213> Artificial Sequence
<220>
<221>
<222>
<223> A part of human DNase gamma
<400> 17
Lys Glu Gln Tyr Ala Phe Leu Tyr Lys Cys
1               5                   10